# EUROPEAN PATENT APPLICATION

(11) **EP 2 508 257 A1**
(43) Date of publication of application: **10.10.2012**
(21) Application number: 10834544.8
(22) Date of filing: 30.11.2010
(51) Int. Cl.: B01J 35/02, A61L 9/00, A61L 9/01

(54) **ANTIVIRAL AGENT, AND ANTIVIRAL AGENT FUNCTIONAL PRODUCT USING SAME**

(30) Priority: 01.12.2009 JP 2009273227
(71) Applicant: Sumitomo Chemical Co., Ltd, Chuo-ku, Tokyo 104-8260 (JP)
(72) Inventor: SUYASU, Yuko, Niihama-shi Ehime 792-0017 (JP); SAKATANI, Yoshiaki, Niihama-shi Ehime 792-0025 (JP); TAKEHARA, Kazuaki, Hachioji-shi, Tokyo 193-0826 (JP)
(74) Representative: Vossius & Partner
(86) International application number: PCT/JP2010/071348
(87) International publication number: WO 2011/068094

(57) **Abstract**

An object of the present invention is to provide an antiviral agent which exhibits high antiviral activity even under irradiation with visible light and is capable of detoxifying a virus. The antiviral agent according to the present application is composed of a dispersion containing noble metals and photocatalyst particles as supported by the noble metals, the dispersion containing 0.01 part by mass to 1 part by mass of noble metal atoms based on 100 parts by mass of photocatalyst particles.

## Description

### Technical Field

The present invention relates to an antiviral agent composed of a dispersion of noble metal-supported photocatalyst, and an antiviral agent functional product using the same.

### Background Art

When a semiconductor is irradiated with light having energy larger than or equal to that of a bandgap thereof, electrons in a valence band are excited to a conduction band to generate holes in the valence band. Since holes thus generated have a strong oxidizing power and electrons thus excited have a strong reducing power, respectively, they exhibit an oxidation-reduction reaction for a substance in contact with the semiconductor. This oxidation-reduction reaction enables formation of active oxygen species including OH radicals, and the active oxygen species can detoxify a virus. The semiconductor capable of exhibiting such a reaction is referred to as a photocatalyst.

It has been known that an antiviral agent in which a photocatalyst of spear-shaped crystal particle and a photocatalyst of sphere-shaped crystal particle are combined by an OH group, detoxify bird influenza virus under irradiation with light emitted from a fluorescent lamp (Patent Literature 1).

Patent Document 1: JP 2008-44869 A

### Disclosure of the Invention

### Problems to be Solved by the Invention

However, since a small amount of OH radicals are formed under visible light irradiation in an antiviral agent containing such a conventional photocatalyst, it was necessary to carry out light irradiation for at least 24 hours or more so as to develop antiviral activity. Moreover, a fluorescent lamp is often used in a state of fitting an acrylic cover containing an ultraviolet absorber indoors. Under such environment, antiviral activity further deteriorated.

Therefore, there has been required an antiviral agent which exhibits high antiviral activity even under irradiation with light emitted from a UV cut fluorescent lamp.
An object of the present invention is to provide an antiviral agent which develops high antiviral activity and is capable of detoxifying a virus even under visible light irradiation.

### Means for Solving the Problems

Therefore, the present inventors have intensively studied so as to develop an antiviral agent which exhibits high activity and found that an antiviral agent including a dispersion of noble metal-supported photocatalyst particles, which contains noble metal atoms in the amount of 0.01 part by mass to 1 part by mass based on 100 parts by mass of photocatalyst particles, exhibits high antiviral activity. Thus, the present invention has been completed.
Accordingly, the antiviral agent according to the present invention includes a dispersion containing noble metals and photocatalyst particles as supported by the noble metals, the dispersion containing 0.01 part by mass to 1 part by mass of noble metal atoms based on 100 parts by mass of photocatalyst particles.

### Effects of the Invention

The antiviral agent of the present invention is composed of a dispersion of noble metal-supported photocatalyst particles in which noble metal-supported photocatalyst particles are dispersed highly and stably, and is easy to apply onto a substrate. Therefore, it is possible to form a photocatalyst layer having uniform film quality on a substrate, and also the photocatalyst layer exhibits high antiviral activity under a practical light source such as visible light included in a fluorescent lamp.
Therefore, according to the present invention, it is possible to provide an antiviral agent which develops high antiviral activity even under visible light irradiation and is capable of detoxifying a virus.

### Best Mode for Carrying Out the Invention

The antiviral agent of the present invention is composed of a dispersion of noble metal-supported photocatalyst particles in which noble metal-supported photocatalyst particles are dispersed in a solvent.

### (Photocatalyst Particles)

Photocatalyst particles to be used in the present invention refer to particulate photocatalyst. Examples of the photocatalyst include compounds of metal elements with oxygen, nitrogen, sulfur and fluorine. Examples of the metal element include Ti, Zr, Hf, V, Nb, Ta, Cr, Mo, W, Mn, Tc, Re, Fe, Co, Ni, Ru, Rh, Pd, Os, Ir, Pt, Cu, Ag, Au, Zn, Cd, Ga, In, Tl, Ge, Sn, Pd, Bi, La and Ce. Examples of the compound include one or more oxides, nitrides, sulfides, acid nitrides, acid sulfides, nitrofluorides, acid fluorides, acid nitrofluorides and the like of these metal elements. Among these compounds, tungsten oxide is suitable for the present invention since it exhibits high antiviral activity when irradiated with visible light (having a wavelength of about 400 nm to about 800 nm).

The size of photocatalyst particles is usually from 40 nm to 250 nm in terms of an average dispersed particle diameter. It is preferred that dispersion stability in a dispersion medium is improved and precipitation of photocatalyst particles can be suppressed as the particle diameter decreases. For example, the particle diameter is preferably 150 nm or less.

Among these photocatalyst particles, tungsten oxide particles can be obtained, for example, by (i) a method in which tungstic acid is obtained as a precipitate by adding an acid to an aqueous solution of a tungstate and the obtained tungstic acid is calcined, (ii) a method in which ammonium metatungstate or ammonium paratungstate are thermally decomposed by heating; or (iii) a method in which metal-like tungsten particles are calcined.

### (Precursor of Noble Metal)

A precursor capable of dissolving in a dispersion medium is used as the precursor of a noble metal to be used in the present invention. When such a precursor is dissolved in a medium, a noble metal element constituting the precursor usually becomes a positively-charged noble metal ion which is present in the dispersion medium. Then, the noble metal ion is reduced to a zero-valent noble metal by irradiation with light, and the noble metal is supported on a surface of photocatalyst particles. Examples of the noble metal include Cu, Pt, Au, Pd, Ag, Ru, Ir and Rh. Examples of the precursor of the noble metal include hydroxides, nitrates, sulfates, halides, organic acid salts, carbonates, phosphates of these noble metals. Among them, the noble metal is preferably Cu, Pt, Au or Pd in view of obtaining high antiviral activity, and Pt is particularly preferable.

Examples of the precursor of Cu include copper nitrate (Cu(NO₃)₂), copper sulfate (CuSO₄), copper chloride (CuCl₂, CuCl), copper bromide (CuBr₂, CuBr), copper iodide (CuI), copper iodate (CuI₂O₆), ammonium copper chloride (Cu(NH₄)2Cl₄), copper oxychloride (Cu₂Cl (OH)₃), copper acetate (CH₃COOCu, (CH₃COO)₂Cu) , copper formate ((HCOO)₂Cu), copper carbonate (CuCO₃), copper oxalate (CuC₂O₄), copper citrate (Cu₂C₆H₄O₇) and copper phosphate (CuPO₄).

Examples of the precursor of Pt include platinum chloride (PtCl₂, PtCl₄), platinum bromide (PtBr₂, PtBr4₎ , platinum iodide (PtI₂, PtI₄), potassium chloroplatinate (K₂(PtCl₄)), hexachloroplatinic acid (K₂PtCl₆), hexachlorplatinic acid (H₂PtCl₆), platinum sulfite (H₃Pt (SO₃)₂OH), tetraammine platinum chloride (Pt(NH₃)₄Cl₂), tetraammine platinum hydrogencarbonate (C₂H₁₄N₄O₆Pt), tetraammine platinum hydrogenphosphate (Pt(NH₃)₄HPO₄), tetraammine platinum hydroxide (Pt (NH₃)₄(OH)₂), tetraammine platinum nitrate (Pt (NO₃) ₂ (NH₃) ₄) , tetraammine platinum tetrachloroplatinum ((Pt(NH₃)₄)(PtCl₄)) and dinitrodiammine platinum (Pt (NO₂)₂(NH₃)₂).

Examples of the precursor of Au include gold chloride (AuCl) , gold bromide (AuBr) , gold iodide (AuI), goldhydroxide (Au(OH)₂), tetrachloroauric acid (HAuCl₄), potassium tetrachloroaurate (KAuCl₄) and potassium tetrabromoaurate (KAuBr₄).

Examples of the precursor of Pd include palladium acetate ((CH₃COO)₂Pd), palladium chloride (PdCl₂), palladium bromide (PdBr₂), palladium iodide (PdI₂), palladium hydroxide (Pd(OH)₂), palladium nitrate (Pd(NO₃)₂), palladium sulfate (PdSO₄), potassium tetrachloropalladate (K₂(PdCl₄)), potassium tetrabromopalladate (K₂(PdBr₄)), tetraammine palladium chloride (Pd(NH₃)₄Cl₂), tetraammine palladium bromide (Pd(NH₃)₄Br₂), tetraammine palladium nitrate (Pd(NH₃)₄(NO₃)₂), tetraammine palladium tetrachloropalladic acid ((Pd(NH₃)₄)(PdCl₄)) and ammonium tetrachloropalladate ((NH₄)₂PdCl₄).

The precursor of a noble metal may be used alone, or two or more kinds of them may be used in combination. The amount of the precursor used is usually 0.01 part by mass or more in terms of a noble metal atom, in view of obtaining a sufficient improving effect of a photocatalytic action, and usually 1 part by mass or less in view of obtaining an effect worth the costs, preferably from 0.05 part by mass to 0.6 part by mass, and more preferably from 0.05 part by mass to 0.2 part by mass, based on 100 parts by mass of photocatalyst particles used.

### (Amount of Radicals Formed)

The dispersion of the noble metal-supported photocatalyst to be used in the present invention forms 4.0 × 10¹⁷ or more OH radicals, preferably 6.0 × 10¹⁷ or more OH radicals, and more preferably 7.5 × 10¹⁷ or more OH radicals, per g of photocatalyst particles by carrying out visible light irradiation. When the amount of OH radicals formed is less than 4.0 × 10¹⁷, sufficient antiviral activity may not be sometimes obtained under visible light irradiation.

In the present invention, the amount of radicals formed is determined by the following procedure. That is, a dispersion of a noble metal-supported photocatalyst is irradiated with visible light in the presence of DMPO (5,5-dimethyl-1-pyrroline-N-oxide) as a radical scavenger and an ESR spectrum is measured, and then an area value of a signal with respect to the obtained spectrum is determined and the amount of radicals formed is calculated from the area value.

In case of calculating the number of radicals, visible light irradiation is carried out at room temperature in atmospheric air at an illuminance of 20, 000 lux for 20 minutes, using a white light-emitting diode as a light source.

The measurement of the ESR spectrum is carried out after irradiating a dispersion of a noble metal-supported photocatalyst with visible light for 20 minutes in a state where of being irradiated with light having an illuminance of less than 500 lux of a fluorescent lamp, as indoor light, using "EMX-Plus" (manufactured by BRUKER). The measurement of the ESR spectrum is carried out under the following measurement conditions:
Temperature: room temperature,
Pressure: atmospheric pressure,
Microwave frequency: 9.86 GHz,
Microwave power: 3.99 mW,
Center field: 3,515 G,
Sweep width: 100 G,
Conv. time: 20.00 mSec,
Time const.: 40.96 ms,
Resolution: 6,000,
Mod. amplitude: 2 G,
Number of scans: 1,
Measurement range: 2.5 cm, and
Tesla meter: Tesla meter is used.

In case of calculating the number of radicals, the calculation is carried out by comparing an ESR spectrum of DMPO-OH as an OH radical adduct of DMPO with an ESR spectrum of a substance in which the number of radicals has been known.

Specifically, the calculation is carried out by the following procedures (1) to (7).

In order to calculate the number of DMPO-OH, a relational equation of the area determined from the ESR spectrum and the number of radical species is determined by the following procedures, first. As the substance in which the number of radicals has been known, 4-hydroxy-TEMPO is used.
(1) 4-Hydroxy-2,2,6,6-tetramethyl-piperidine-1-oxyl (4-hydroxy-TEMPO) (having a purity of 98%, 0.17621 g) is dissolved in 100 mL of water. The obtained solution is designated as a solution A.
(2) To 1 mL of the solution A, water is added to make 100 mL. The obtained solution is designated as a solution B.
(3) To 1 mL of the solution B, water is added to make 100 mL. The obtained solution is designated as a solution C. The concentration of the solution is 0.001 mM.
(4) To 1 mL of the solution B, water is added to make 50 mL. The obtained solution is designated as a solution D. The concentration of the solution is 0.002 mM.
(5) To 3 mL of the solution B, water is added to make 100 mL. The obtained solution is designated as a solution E. The concentration of the solution is 0.003 mM.
(6) Each of the solutions C, D and E is filled in a flat cell and the measurement of an ESR spectrum is carried out. One area at a low magnetic field side of the obtained three peaks (area ratio: 1:1:1) is determined and an area obtained by tripling the obtained one area is regarded as an area in each concentration of 4-hydroxy-TEMPO. The area of a peak is obtained by converting an ESR spectrum (differential-type) to an integral-type one.
(7) Since 4-hydroxy-TEMPO has one radical per one molecule, the number of radicals of 4-hydroxy-TEMPO contained in solutions C to E is calculated, and a first-order linear approximate equation can be obtained by using the number of radicals and the area determined from the ESR spectrum.

Next, the number y1 of OH radicals after irradiation with a white light-emitting diode is calculated from an ESR spectrum of DMPO-OH and the first-order linear approximate equation calculated using 4-hydroxy-TEMPO having a known concentration. Furthermore, the number y2 of OH radicals contained in a dispersion of noble metal-supported photocatalyst particles before light irradiation is calculated in the same manner. A difference between them (y1 - y2) is the number of OH radicals formed by irradiation with a white light-emitting diode.

### (Raw Dispersion)

In the present invention, a raw dispersion is used in which the above photocatalyst particles are dispersed and the above precursor of a noble metal is dissolved in a dispersion medium.

### (Dispersion Medium)

There is no particular limitation on a dispersion medium, and an aqueous solvent containing water as a main component is usually used. Specifically, the dispersion medium may be either water alone, or a mixed solvent of water and a water-soluble organic solvent. In case of using the mixed solvent of water and a water-soluble organic solvent, the content of water is preferably 50% by mass or more. Examples of the water-soluble organic solvent include water-soluble alcohol solvents such as methanol, ethanol, propanol and butanols; acetone, methyl ethyl ketone, ethyl cellosolve, butyl cellosolve, ethyl acetate, diethylether and the like. The dispersion medium may be used alone, or two or more kinds of them may be used in combination. The amount of the dispersion medium is usually 5-fold by mass to 200-fold by mass based on photocatalyst particles. When the amount of the dispersion medium is less than 5-fold by mass, photocatalyst particles are likely to be precipitated. In contrast, when the amount of the dispersion medium exceeds 200-fold by mass, it is disadvantageous in respect of volume efficiency.

### (Sacrificial Agent)

In the present invention, a sacrificial agent is added to a raw dispersion. For example, alcohols such as ethanol, methanol and propanol; ketones such as acetone; and carboxylic acids such as oxalic acid are used as the sacrificial agent. When the sacrificial agent is a solid, the sacrificial agent may be used after dissolving it in a suitable solvent, or the sacrificial agent may be used as it is. The sacrificial agent may be added to a raw dispersion after a certain period of time of light irradiation, and further light irradiation may be carried out thereafter. The amount of the sacrificial agent is usually 0.001-fold by mass to 0.3-fold by mass, preferably 0.005-fold by mass to 0.1-fold by mass, based on the dispersion medium. When the amount of the sacrificial agent used is less than 0.001-fold by mass, the support of a noblemetal onto photocatalyst particles is insufficient. In contrast, when the amount exceeds 0.3-fold by mass, the amount of the sacrificial agent is excessive and does not give an effect which is worth the costs.

### (Preparation of Raw Dispersion)

A raw disper sionmay be prepared by dispersing photocatalyst particles in a dispersion medium. In case of dispersing photocatalyst particles in the dispersion medium, a dispersion treatment is preferably carried out using a known device such as a wet stirred media mill.

### (Irradiation with Light)

In the present invention, such a raw dispersion is irradiated with light. The light irradiation toward a raw dispersion may be carried out with stirring. The dispersion may be allowed to pass through a tube made of a transparent glass or plastic, and light may be irradiated from the inside or outside of the tube. There is no particular limitation on a light source as long as it can emit light having energy larger than or equal to that of a bandgap of photocatalyst particles. Specifically, a germicidal lamp, a mercury lamp, a luminescent diode, a fluorescent lamp, a halogen lamp, a xenon lamp and sunlight can be used. The wavelength of light for irradiation is usually from 180 nm to 500 nm. The light irradiation time is usually 20 minutes or more, preferably 1 hour or more, and usually 24 hours or less, preferably 6 hours or less before and after the addition of the sacrificial agent, since a sufficient amount of a noble metal can be supported. When the irradiation time exceeds 24 hours, an effect which is worth the costs of the light irradiation can not be obtained since, by that time, most precursors of a noble metal are converted to the noble metal which is supported on photocatalyst particles. When the light irradiation is not carried out before the addition of the sacrificial agent, supporting of the noble metal to photocatalyst particles becomes un-uniform and thus high antiviral activity cannot be obtained.

### (pH Adjustment)

In the present invention, the light irradiation is carried out while maintaining the pH of a raw dispersion in a range from 2.5 to 4.5, and preferably from 2.8 to 4.0. Usually, the pH of the dispersion gradually changes to an acidic pH when a noble metal is supported on a surface of photocatalyst particles by light irradiation. Accordingly, a base may be added to the dispersion in order to maintain the pH in a range defined in the present invention. Thereby, a dispersion of a noble metal-supported photocatalyst, which is excellent in dispersibility, is obtained. Examples of the base include aqueous solutions of ammonia, sodium hydroxide, potassium hydroxide, magnesium hydroxide, calcium hydroxide, strontium hydroxide, barium hydroxide and lanthanum hydroxide. Among these bases, ammonia water and sodium hydroxide are preferably used.

### (Amount of Dissolved Oxygen)

In the present invention, if necessary, the amount of oxygen dissolved in a raw dispersion is adjusted to 1.0 mg/L or less, and preferably 0.7 mg/L or less, before or during irradiation of the low dispersion with light. The amount of dissolved oxygen can be adjusted by blowing an oxygen-free gas into a raw dispersion, and examples of the gas include nitrogen and noble gases (helium, neon, argon, krypton, etc.). When the amount of dissolved oxygen exceeds 1.0 mg/L, a reductive reaction of dissolved oxygen occurs in addition to supporting of a precursor of a noble metal, and thus supporting the noble metal becomes un-uniform and high antiviral activity cannot be obtained.

### (Noble Metal-Supported Photocatalyst)

While adjusting the pH of a raw dispersion, the amount of dissolved oxygen is adjusted to a predetermined value or less and the raw dispersion is subjected to light irradiation. After addition of a sacrificial agent and further light irradiation, a noble metal precursor is converted to a noble metal which is supported on a surface of photocatalyst particles, and thus the objective noble metal-supported photocatalyst particles can be obtained. The obtained noble metal-supported photocatalyst particles are dispersed in a dispersion medium used without being precipitated.

### (Dispersion of Noble Metal-Supported Photocatalyst Particles)

The obtained dispersion of noble metal-supported photocatalyst particles in which noble metal-supported photocatalyst particles are dispersed is easy to handle since it is excellent in dispersibility of noble metal-supported photocatalyst particles, and also develops high antiviral activity.

The dispersion of noble metal-supported photocatalyst particles may contain various known additives as long as they do not impair the effects of the present invention. Examples of additives include silicon compounds such as amorphous silica, silica sol, water glass, alkoxysilane and organopolysiloxane; aluminum compounds such as amorphous alumina, alumina sol and aluminum hydroxide; aluminosilicates such as zeolite and kaolinite; alkaline earth metal oxides such as magnesium oxide, calcium oxide, strontium oxide and barium oxide; alkaline earth metal hydroxides such as magnesium hydroxide, calcium hydroxide, strontium hydroxide and barium hydroxide; hydroxides or oxides of metal elements such as Ti, Zr, Hf, V, Nb, Ta, Cr, Mo, W, Mn, Tc, Re, Fe, Co, Ni, Ru, Rh, Os, Ir, Ag, Zn, Cd, Ga, In, Tl, Ge, Sn, Pb, Bi, La and Ce; zirconium oxalate, calcium phosphate, molecular sieves, activated charcoal, polycondensates of organic polysiloxane compounds, phosphates, fluorinated polymers, silicon-based polymers, acrylic resins, polyester resins, melamine resins, urethane resins and alkyd resins. When these additives are used, they may be used alone, or two or more kinds of them may be used in combination.

It is also possible to use, as the additive, binders for retaining photocatalyst particles more firmly on a surface of a substrate in case of forming a photocatalyst layer on the surface of the substrate using a photocatalyst (see, for example, JP 8-67835 A, JP 9-25437 A, JP 10-183061 A, JP 10-183062 A, JP 10-168349 A, JP 10-225658 A, JP 11-1620 A, JP 11-1661 A, JP 2002-80829 A, JP 2004-059686 A, JP 2004-107381 A, JP 2004-256590 A, JP 2004-359902 A, JP 2005-113028 A, JP 2005-230661 A, JP 2007-161824 A, WO 96/029375, WO 97/000134, WO 98/003607, etc.).

### (Antiviral Agent Functional Product)

The antiviral agent functional product of the present invention includes a photocatalyst layer formed by using a dispersion of noble metal-supported photocatalyst particles on a surface. Herein, the photocatalyst layer can be formed, for example, by a conventionally known film formation method in which a dispersion of noble metal-supported photocatalyst particles of the present invention is applied onto a surface of a substrate (product) and then a dispersion medium is vaporized. There is no particular limitation on the thickness of the photocatalyst layer. Usually, the thickness may be appropriately set in a range from several hundreds nm to several mm according to applications thereof. The photocatalyst layer may be formed at any part as long as the part is an inner or outer surface of a substrate (product). For example, the photocatalyst layer is preferably formed on a surface which is irradiated with light (visible light) and is also specially connected continuously or intermittently with the place where malodorous substances are generated, or the place where pathogenic bacteria exist. There is no particular limitation on the material of the substrate (product) as long as it can retain the photocatalyst layer with the strength which can endure practical use, and the objective product includes products made of every material, for example, as plastics, metals, ceramics, woods, concretes and papers.

Specific examples of the antiviral agent functional product of the present invention include a surface of substrates which are in contact with unspecified number of peoples, for example, building materials such as ceiling materials, tiles, glasses, wall papers, wall materials and floors; automotive interior materials (automotive instrument panels, automotive sheets, automotive ceiling material, automotive glasses); household electrical appliances such as refrigerators and air conditioners; textile products such as clothings and curtains; and buttons of elevators and straps in a train.

The antiviral agent functional product of the present invention exhibits high antiviral activity by light irradiation even in an indoor environment where only light from visible light sources such as a fluorescent lamp, a sodium lamp and a light-emitting diode, needless to say in an outdoor environment. Accordingly, when a dispersion of noble metal-supported photocatalyst particles of the present invention is applied onto a surface of substrates which are in contact with unspecified number of peoples, for example, building materials such as ceiling materials, tiles, glasses, wall papers, wall materials and floors; automotive interior materials (automotive instrument panels, automotive sheets, automotive ceiling material); household electrical appliances such as refrigerators and air conditioners; furnitures such as desks, chairs, tables, chests and cabinets; and textile products such as clothings and curtains; and then dried, it is possible to detoxify meleagrid herpesvirus, Marek's disease virus, infectious bursal disease virus, Newcastle disease virus, infectious bronchitis virus, infectious laryngotracheitis virus, avian encephalomyelitis virus, Chicken anaemia virus, fowlpox virus, avian reovirus, avian leukosis virus, reticuloendotheliosis virus, avian adenovirus and hemorrhagic enteritis virus, herpesvirus, smallpox virus, cowpox virus, measles virus, adenovirus, Coxsackie virus, calicivirus, retrovirus, coronavirus, bird influenza virus, human influenza virus, swine influenza virus, norovirus, foot and mouth disease virus, and recombinants thereof, by irradiation with light emitted from interior lighting.

### Examples

The present invention will be described in more detail below by way of Examples, but the present invention is not limited thereto.
The measuring methods in the respective Examples are as follows.

### 1. BET Specific Surface Area

BET specific surface area of photocatalyst particles was measured by a nitrogen adsorption method using a specific surface area measuring instrument ("MONOSORB", manufactured by Yuasa Ionics Co., Ltd.).

### 2. Average Dispersed Particle Diameter (nm)

Using a submicron particle size distribution analyzer ("N4Plus", manufactured by Coulter Corporation), particle size distribution was measured, and the results obtained by automatic monodisperse mode analysis using software attached to the analyzer were employed as an average dispersed particle diameter.

### 3. Crystalline Type

X-ray diffraction spectrum was measured by using an X-ray diffractometer ("RINT2000/PC", manufactured by Rigaku Corporation) and the crystalline type (crystal structure) was determined from the spectrum.

### 4. Amount of Dissolved Oxygen

The amount of oxygen dissolved in a raw dispersion was measured by using a dissolved oxygen meter ("OM-51", manufactured by HORIBA, Ltd.).

### 5. Measurement of Amount of OH Radicals Formed

In a sample tube (having a capacity of 13.5 mL, and measuring 2 cm in inner diameter and 6.5 cm in height (height of a content fluid fillable portion of 5.5 cm)), a stirrer and 2 mL of a dispersion of noble metal-supported photocatalyst particles (containing 2 mg of noble metal-supported photocatalyst particles) adjusted to a concentration of 0.1% by mass with water were placed, and then 23 µL of DMPO (having a purity of 97%) was charged so that the concentration becomes 100 mM. After stirring with a stirrer, the supernatant was injected into a flat cell and ESR was measured. The obtained one was employed as a sample for light irradiation of 0 minute. Next, using a white light-emitting diode (LED bed lamp "LEDA-21002W-LS1" (corresponding to white color) having a main wavelength of about 450 nm, manufactured by Toshiba Lighting & Technology Corporation), the sample tube was irradiated with light from above the sample tube for 20 minutes. An illuminance at a liquid level in the sample tube was 20,000 lux (measured by an illuminometer "T-10" manufactured by Minolta Co., Ltd.). Then, the supernatant was placed in a flat cell and ESR of the thus formed DMPO-OH adduct was measured. The number of OH radicals formed by visible light irradiation was calculated from the number of DMPO-OH adducts at a light irradiation time of 0 minute and 20 minutes, and then the amount of OH radicals formed per g of noble metal-supported photocatalyst particles was determined from the number of OH radicals and the weight (2 mg) of noble metal-supported photocatalyst particles.

### 6. Determination of Antiviral Activity

Antiviral activity was evaluated by measuring virus titers of influenza virus and adenovirus under visible light irradiation using a fluorescent lamp. More specifically, the obtained dispersion of noble metal-supported photocatalyst particles was uniformly applied onto one surface of a glass plate measuring 5 cm × 5 cm × 2 mm in a coating weight in terms of the solid content per unit area of 1 g/m² using a spin coater ("1H-D7", manufactured by MIKASA). Then, the glass plate was dried by maintaining in an oven at 130°C for 10 minutes in atmospheric air to obtain a photocatalyst layer on one surface of the glass plate. The photocatalyst layer was irradiated with ultraviolet light from a black light at a ultraviolet intensity of 2 mW/cm² (measured by an ultraviolet intensity meter "UVR-2" manufactured by TOPCON CORPORATION with a light receiving section "UD-36" manufactured by the same company attached thereto) for 16 hours to obtain a sample for the measurement of antiviral activity.

Using the sample for the measurement of antiviral activity, evaluation was carried out by the method according to "10. Film Adhesion Method" in Japanese Industrial Standards JIS R1702 (2006) "Fine Ceramics - Test Method for Antibacterial Activity of Photocatalytic Products under Photoirradiation and Efficacy."

### 6-1 Influenza Virus

As influenza virus, A/Northern pintail/Miyagi/1472/08 (H1N1) was used. More specifically, the photocatalyst layer of the sample was inoculated with a test medium containing the virus, covered with a covering film and closely attached with it, and stored under visible light irradiation or under the condition of light shielding at a room temperature of 25±5°C for 6 hours before virus titer per one test sample was determined as the logarithm value of 50% tissue culture infectious dose (LogTCID50/ml) using MDCK cells (Madin-Darby canine kidney cell, a cell strain derived from dog kidney tubule epidermal cells) . The test liquid containing virus and the MDCK cell were prepared in the following maintenance medium (MM) or growth medium (GM).

A medium for preparing virus or culturing cells was prepared as follows: 9.4 g of Eagle's minimum essential medium (Nissui Pharmaceutical Co., Ltd., Tokyo) and 3.0 g of Tryptose Phosphate Broth (TPB, Difco laboratories, Detroit, MI, USA) were dissolved in 1,000 ml of distilled water and sterilized by a process of steaming under pressure, and then 7% NaHCO₃, 200 mM of L-glutamine, penicillin and streptomycin were added in each final concentration of 2%, 1%, 100 units/ml and 100 µg/ml, respectively, to obtain a maintenance medium (hereinafter referred to as MM). Further, fetal calf serum (hereinafter referred to as FCS) was added to MM at a rate of 5% to obtain a growth medium (hereinafter referred to as GM). The MDCK cells (1.0 × 10⁵ cells/ml) were inoculated into wells of a 96-well tissue culture plate in each dose of 200 µl to be incubated in a carbon dioxide incubator at 37°C.
The virus was inoculated in 10 day old embryonated chicken eggs by the allantoic cavity route to recover the allantoic fluid 3 days after. After the recovered affected allantoic fluid was ultra-centrifuged at 25,000 rpm, 4 °C for 90 minutes, the obtained pellet was resuspended in 1/100 parts of phosphate buffered saline (PBS) to the initial allantoic fluid to prepare a virus fluid. The virus fluid was 100-fold diluted with PBS to add it dropwise into the photocatalytic produc ts in an amount of 100 µl to determine its Antiviral activity in the FilmAdhesion Method. After a given period of time, the glass plate and the film were packed in a plastic bag to add 1 ml of MM into the bag to recover the virus fluid. The recovered virus fluid was transferred into a microtube and centrifuged at 15, 000 rpm for 3 minutes to yield a supernatant to determine a virus titer of the residual virus in it.
To measure the virus titer, each of the virus fluids which were 10-fold serial diluted with MM was inoculated into 4 wells of a 96-well tissue culture plate, respectively. However, before inoculation, MDCK cells were washed with 200 µl each of PBS three times; trypsin was added to MM in an amount of 2 µg/ml to feed them into wells 100 µl each; and then, 100 µl of the diluted virus fluid was inoculated in each well.

Evaluation of the antiviral activity was performed by using three replicated samples to test for antiviral activity simultaneously to yield an average of logarithmic value of the obtained three virus titers. The visible light was irradiated by commercially available white fluorescent lamps (20 W, two lamps) as a light source so that the photocatalyst layer covered with the covering film was irradiated with visible light from the lamps through an acrylic resin plate ("N113", manufactured by NITTO JUSHI KOGYO CO., LTD.). At this time, the illuminance in the vicinity of the coating film was adjusted to 1,000 lux (measured by an illuminometer "T-10", manufactured by Minolta Co., Ltd.). It is possible to say that the smaller the virus titer measured 2 or 6 hours after visible light irradiation, the more the antiviral activity against influenza virus, namely, photocatalytic activity is higher. Measurement limit of the logarithmic value of the virus titer was 1.5.

### 6-2 Adenovirus

As adenovirus, avian adenovirus was used, wherein the standard strain Ote was provided from National Agriculture and Food Research Organization (Ibaragi). The avian adenovirus was passage cultured through chicken kidney cultured cells (CK cells) described below to result in an affected broth which was used as a virus fluid, divided and then stored at -80°C before use.

The photocatalyst layer was inoculated with the test medium containing virus, covered with the covering film and closely adhered with it, and then stored under visible light irradiation or under a condition of light shielding at a room temperature, 25±5°C for 6 hours to determine virus titer per one test sample by the plaque forming test on the below-mentioned CK cell. However, onto the photocatalyst layer, 100 µL of the test medium containing 1,000-fold diluted virus with redistilled water (hereinafter referred to as dW2) was added dropwise to test it in the maintenance medium (hereinafter referred to as MM) until the recovered fluid was to be 1,000 µL.

### (Chicken Kidney Cultured Cells)

After a day-old chick (Koiwai Farm) was euthanized with carbon dioxide gas, a kidney taken from it was digested with a trypsin solution to obtain chicken kidney (hereinafter referred to as CK) cells. The CK cells (cell concentration: 0.3%) were inoculated in Tissue Culture Dishes (PS, 60 × 15 mm, with vents, sterile, Greener bio-one) in an amount of 4 ml each to be incubated in a 5% CO₂ incubator at 37 °C. However, as the medium used herein, a growth medium for CK (hereinafter referred to as GM for CK) was prepared by dissolving 9.4 g of E-MEM medium and 3.0 g of tryptose phosphate broth (TPB) in 1,000 ml of dW2, sterilizing them by a process of steaming under pressure and then adding 7% NaHCO₃, 200 mM of L-glutamine, Calf Serum (hereinafter referred to as CS), penicillin, streptomycin and amphotericin B thereto in each concentration of 2%, 1%, 5%, 100 units/ml, 100 µg/ml and 0.5 µg/ml, respectively. As a basal medium of an agar overlay medium used for plaque forming test, primary agar overlay MM was prepared by dissolving 9.4 g of E-MEM medium and 3.0 g of TPB in 500 ml of dW2, sterilizing them by a process of steaming under pressure, and then adding 7% NaHCO₃, 200 mM of L-glutamine, penicillin, streptomycin and amphotericin B thereto in each concentration of 2%, 1%, 100 units/ml, 100 µg/ml and 0.5 µg/ml, respectively. Also, secondary agar overlay MM was obtained by further adding 10 ml of 0.5% neutral red to the primary agar overlay MM. Bact Agar (Difco laboratories, Detroit, MI, USA) (1.6 g) was dissolved in 100 ml of dW2 and then sterilized by a process of steaming under pressure to result in 2 × Bact Agar. The primary agar overlay MM and the 2 × Bact Agar which was melted in a microwave oven were mixed in equal amount to prepare a primary agar overlay medium. Also, the secondary agar overlay MM and the 2 × Bact Agar which was melted in a microwave oven were mixed in equal amount to prepare a second agar overlay medium.

### (Plaque Forming Test)

For avian adenovirus, the serial diluted virus was first inoculated in CK cells (100 µl/dish, 2 dishes/sample). One hour after inoculation, the inoculation fluid was removed before 3 ml of the primary agar overlay medium was overlaid thereon. Additional 5 days after overlaying, 3 ml of the secondary agar overlay medium was further overlaid thereon. In the end, seven days after inoculation, by counting the number of plaques formed, it was recorded as the number of plaque forming units (hereinafter referred to as PFU/ml). Detection limit of the virus titer of avian adenovirus was determined as 10⁵ PFU/ml.

Evaluation of the antiviral activity was performed by using two replicated samples to test for antiviral activity simultaneously to yield an average of logarithmic value of the obtained two virus titers. Visible light was irradiated by commercially available white fluorescent lamps (20 W, two lamps) as a light source so that the photocatalyst layer covered with the covering film was irradiated with visible light from the lamps through an acrylic resin plate (NITTO JUSHI KOGYO CO., LTD., "N113"). At this time, the illuminance measured in the vicinity of the coated film was adjusted to 1,000 lux (measured by an illuminometer "T-10", manufactured by Minolta Co., Ltd.). It is possible to say that the smaller the virus titer measured 6 hours after visible light irradiation, the more the antiviral activity against influenza virus, namely, photocatalytic activity is higher. Measurement limit of the logarithmic value of the virus titer was 5.2.

### (Example 1)

To 4 kg of ion-exchange water as a dispersion medium, 1 kg of tungsten oxide particles (manufactured by NIPPON INORGANIC COLOUR & CHEMICAL CO., LTD.) were added, followed by mixing to obtain a mixture. The obtained mixture was subjected to a dispersion treatment using a wet stirred media mill to obtain a dispersion of tungsten oxide particles.

Tungsten oxide particles in the obtained dispersion of tungsten oxide particles had an average dispersed particle diameter of 118 nm. The dispersion of tungsten oxide particles was partially vacuum-dried to obtain a solid component. As a result, the obtained solid component had a BET specific surface area of 40 m²/g. In the same manner, the mixture before a dispersion treatment was vacuum-dried to obtain a solid component, and then an X-ray diffraction spectrum of the solid component of the mixture before a dispersion treatment and that of the solid component after a dispersion treatment were respectively measured and compared. As a result, the solid components showed the same peak shape, and a change in crystalline type due to a dispersion treatment was not recognized. At this point, the obtained dispersion of tungsten oxide particles was left to stand at 20°C for 24 hours. As a result, solid-liquid separation was not recognized during storage.

To the dispersion of tungsten oxide particles, an aqueous solution of hexachlorplatinic acid (H₂ PtCl₆) was added so that hexachloroplatinic acid exists in the amount of 0.12 part by mass in terms of platinum atom based on 100 parts by mass of tungsten oxide particles to obtain a hexachlorplatinic acid-containing dispersion of tungsten oxide particles as a raw dispersion. The solid component (amount of tungsten oxide particles) contained in 100 parts by mass of the raw dispersion was 17.6 parts by mass (concentration of the solid component was 17.6% by mass). The pH of the raw dispersion was 2.0.

Using a light irradiation apparatus including a pH electrode, a pH controller (set to pH 3.0) which is connected to the pH electrode and also has a control mechanism of adjusting the pH to a given value by supplying 0.1% by mass ammonia water, and a glass tube (measuring 37 mm in inner diameter and 360 mm in height) which is equipped with a nitrogen blowing tube and is also provided with a double-tube germicidal lamp ("GLD15MQ", manufactured by SANYO DENKI CO., LTD.), the pH of a raw dispersion was adjusted to pH 3.0 while circulating 1,200 g of the raw dispersion at a rate of 1 L per minute. Nitrogen was blown at a rate of 2 L per minute. After the amount of oxygen dissolved in the raw dispersion became 0. 5 mg/L, nitrogen was subsequently blown and light irradiation (irradiation with ultraviolet light) was carried out for 2 hours while circulating the raw dispersion. Furthermore, methanol was added so that the concentration thereof becomes 1% by mass based on the entire sol vent, and then nitrogen was blown and light irradiation was carried out for 3 hours while circulating the raw dispersion to obtain a dispersion of platinum-supported tungsten oxide particles. The total amount of 0.1% by weight ammonia water consumed before light irradiation and during light irradiation was 103 g. During light irradiation, the pH was constant at 3.0.

The obtained dispersion of platinum-supported tungsten oxide particles was left to stand at 20°C for 24 hours. As a result, solid-liquid separation was not observed after storage. The amount of OH radicals formed under white light-emitting diode irradiation of the dispersion of platinum-supported tungsten oxide particles was measured. As a result, it was found that 8.5 × 10¹⁷ OH radicals were formed per g of platinum-supported tungsten oxide particles. Next, antibacterial activity of a photocatalyst layer formed by using the dispersion of platinum-supported tungsten oxide particles was evaluated.

In the photocatalyst layer of the present invention, the virus titter against influenza virus, which was 6.8 when the light irradiation time is 0 hour, became less than 1.5 (less than detection limit) measured 2 hours after light irradiation. When the photocatalyst layer was left to stand in the dark for 2 hours without carrying out light irradiation, the virus titer became 4.8.

Furthermore, in the photocatalyst layer of the present invention, the virus titer against fowl adenovirus, which was 8.9 when the light irradiation time is 0 hour, became less than 5.2 (less than detection limit) measured 6 hours after light irradiation.

### (Comparative Example 1)

In the same manner as in Example 1, except that unprocessed glass with no photocatalyst layer formed thereon was used, antiviral activity was evaluated. As a result, the virus titer against influenza virus, which was 6.8 when the light irradiation time is 0 hour, was less than 6.8 measured 2 hours after light irradiation. Even when the photocatalyst layer was left to stand in the dark for 2 hours without carrying out light irradiation, the virus titer was also 6.8.

The virus titer against fowl adenovirus, which was 8.9 when the light irradiation time is 0 hour, became 7.9 measured 6 hours after light irradiation.

### (Comparative Example 2)

The amount of OH radicals formed under white light-emitting diode irradiation of the dispersion of tungsten oxide particles (which do not support platinum) was measured, in place of the dispersion of platinum-supported tungsten oxide particles. As a result, it was found that 3.2 × 10¹⁷ OH radicals were formed per g of tungsten oxide particles. Next, antiviral activity of a photocatalyst layer formed by using the dispersion of platinum-supported tungsten oxide particles was evaluated. As a result, the virus titer against influenza virus, which was 7.5 when the light irradiation time is 0 hour, became 4.6 measured 2 hours after light irradiation.

### (Reference Example 1)

The antiviral agent obtained in Example 1 is applied onto a surface of a ceiling material constituting a ceiling and then dried, and thus a photocatalyst layer can be formed on a surface of the ceiling material. Thereby, it is possible to detoxify enveloped viruses such as avian influenza virus, human influenza virus, swine influenza virus and non-enveloped viruses such as adenovirus and aphthovirus in indoor space by irradiating with light emitted from interior lighting.

### (Reference Example 2)

The antiviral agent obtained in Example 1 is applied onto tiles provided on interior wall surface and then dried, and thus a photocatalyst layer can be formed on a surface of tiles. Thereby, it is possible to detoxify enveloped viruses such as avian influenza virus, human influenza virus, swine influenza virus and non-enveloped viruses such as adenovirus and aphthovirus in indoor space by irradiating with light emitted from interior lighting.

### (Reference Example 3)

The antiviral agent obtained in Example 1 is applied onto a surface at the interior side of windowpane and then dried, and thus a photocatalyst layer can be formed on the surface of windowpane. Thereby, it is possible to detoxify enveloped viruses such as avian influenza virus, human influenza virus, swine influenza virus and non-enveloped viruses such as adenovirus and aphthovirus in indoor space by irradiating with light emitted from interior lighting.

### (Reference Example 4)

The antiviral agent obtained in Example 1 is applied onto a wall paper and then dried, and thus a photocatalyst layer can be formed on a surface of the wall paper. Thereby, it is possible to detoxify enveloped viruses such as avian influenza virus, human influenza virus, swine influenza virus and non-enveloped viruses such as adenovirus and aphthovirus in indoor space by irradiating with light emitted from interior lighting.

### (Reference Example 5)

The antiviral agent obtained in Example 1 is applied onto an interior floor and then dried, and thus a photocatalyst layer can be formed on the floor. Thereby, it is possible to detoxify enveloped viruses such as avian influenza virus, human influenza virus, swine influenza virus and non-enveloped viruses such as adenovirus and aphthovirus in indoor space by irradiating with light emitted from interior lighting.

### (Reference Example 6)

The antiviral agent obtained in Example 1 is applied onto a surface of automotive interior materials such as automotive instrument panels, automotive sheets, automotive ceiling materials, and the inside of automotive glasses and then dried, and thus a photocatalyst layer can be formed on a surface of these automotive interior materials. Thereby, it is possible to detoxify enveloped viruses such as avian influenza virus, human influenza virus, swine influenza virus and non-enveloped viruses such as adenovirus and aphthovirus in indoor space by irradiating with light emitted from interior lighting.

### (Reference Example 7)

The antiviral agent obtained in Example 1 is applied onto a surface of an air conditioner and then dried, and thus a photocatalyst layer can be formed on the surface of the air conditioner. Thereby, it is possible to detoxify enveloped viruses such as avian influenza virus, human influenza virus, swine influenza virus and non-enveloped viruses such as adenovirus and aphthovirus in indoor space by irradiating with light emitted from interior lighting.

### (Reference Example 8)

The antiviral agent obtained in Example 1 is applied onto the inside of a refrigerator and then dried, and thus a photocatalyst layer can be formed on the inside of the refrigerator. Thereby, it is possible to detoxify enveloped viruses such as avian influenza virus, human influenza virus, swine influenza virus and non-enveloped viruses such as adenovirus and aphthovirus in the inside of the refrigerator by irradiating with light emitted from interior lighting.

### (Reference Example 9)

The antiviral agent obtained in Example 1 is applied onto a surface of substrates which are in contact with unspecified number of peoples, for example, buttons of elevators and straps in a train and then dried, and thus a photocatalyst layer can be formed on a surface of these substrates. Thereby, it is possible to detoxify enveloped viruses such as avian influenza virus, human influenza virus, swine influenza virus and non-enveloped viruses such as adenovirus and aphthovirus on the substrate surface by irradiating with light emitted from interior lighting.

### (Reference Example 10)

The antiviral agent obtained in Example 1 is applied onto a surface of furnitures such as tables, chests, cabinets and desks and then dried, and thus a photocatalyst layer can be formed on a surface of these substrates. Thereby, it is possible to detoxify enveloped viruses such as avian influenza virus, human influenza virus, swine influenza virus and non-enveloped viruses such as adenovirus and aphthovirus on the substrate surface by irradiating with light emitted from interior lighting.

### (Reference Example 11)

The antiviral agent obtained in Example 1 is applied onto a surface of household electrical appliances such as personal computers, printers, scanners, copying machines, telephones, televisions, stereos, washing machines, stoves, dryers and microwave ovens and then dried, and thus a photocatalyst layer can be formed on a surface of these substrates. Thereby, it is possible to detoxify enveloped viruses such as avian influenza virus, human influenza virus, swine influenza virus and non-enveloped viruses such as adenovirus and aphthovirus on the substrate surface by irradiating with light emitted from interior lighting.

### (Reference Example 12)

The antiviral agent obtained in Example 1 is applied onto a surface of fusmas, tatamis, shojis and the like and then dried, and thus a photocatalyst layer can be formed on a surface of these substrates. Thereby, it is possible to detoxify enveloped viruses such as avian influenza virus, human influenza virus, swine influenza virus and non-enveloped viruses such as adenovirus and aphthovirus on the substrate surface by irradiating with light emitted from interior lighting.

### (Reference Example 13)

The antiviral agent obtained in Example 1 is applied onto a surface of a film and then dried, and thus a photocatalyst layer can be formed on a surface of the film and also the film is provided on building materials, furnitures, walls, floors, ceilings, touch panels, buttons of machineries, doors, doorknobs, hand rails of stairs, inner walls of passenger cabins of aircrafts and trains, and the like. Thereby, it is possible to detoxify enveloped viruses such as avian influenza virus, human influenza virus, swine influenza virus and non-enveloped viruses such as adenovirus and aphthovirus on the film surface by irradiating with light emitted from interior lighting.

## Claims

1. An antiviral agent comprising a dispersion containing noble metals and photocatalyst particles as supprtedby the noble metals, the dispersion containing 0.01 part by mass to 1 part by mass of noble metal atoms based on 100 parts by mass of photocatalyst particles.

2. The antiviral agent according to claim 1, including a dispersion of noble metal-supported photocatalyst particles, the dispersion containing 0.01 part by mass to 1 part by mass of noble metal atoms based on 100 parts by mass of photocatalyst particles,
wherein the dispersion of noble metal-supported photocatalyst particles forms 4.0 × 10¹⁷ or more OH radicals per g of noble metal-supported photocatalyst particles by irradiating with visible light for 20 minutes, using a white light-emitting diode having an illuminance of 20,000 lux as a light source.

3. The antiviral agent according to claim 1 or 2, wherein the noble metal is at least one noble metal selected from the group consisting of Cu, Pt, Au, Pd, Ag, Ru, Ir and Rh.

4. The antiviral agent according to any one of claims 1 to 3, wherein the photocatalyst is tungsten oxide.

5. An antiviral agent functional product comprising a substrate and a photocatalyst layer formed on a surface of the substrate, wherein the photocatalyst layer is formed by using the antiviral agent according to any one of claims 1 to 4.
